# EUROPEAN PATENT SPECIFICATION

(11) **EP 1 634 958 B1**
(45) Date of publication and mention of the grant of the patent: **18.02.2009**
(21) Application number: 04735497.2
(22) Date of filing: 31.05.2004
(51) Int. Cl.: C12P 41/00, C12P 17/06

(54) **METHOD FOR PRODUCING OPTICALLY ACTIVE CHROMAN-CARBOXYLATE**
VERFAHREN ZUR HERSTELLUNG VON OPTISCH AKTIVEM CHROMAN-CARBONSÄUREESTER
PROCEDE DE PRODUCTION DE CHROMAN-CARBOXYLATE OPTIQUEMENT ACTIF

(30) Priority: 04.06.2003 JP 2003159353
(43) Date of publication of application: 15.03.2006
(73) Proprietor: MITSUBISHI GAS CHEMICAL COMPANY, INC., Chiyoda-ku, Tokyo 100-8324 (JP)
(72) Inventor: KYUUKO, Youichi, Mitsubishi Gas Chemical Co., Inc., Tsukuba-shi, Ibaraki 3004247 (JP); KOSHIISHI, Sachiko, Mitsubishi Gas Chem.Co., Inc., Tsukuba-shi, Ibaraki 3004247 (JP); HIDAKA, Toshio, Mitsubishi Gas Chemical Co., Inc., Tsukuba-shi, Ibaraki 3004247 (JP)
(74) Representative: Gille Hrabal Struck Neidlein Prop Roos
(86) International application number: PCT/JP2004/007851
(87) International publication number: WO 2004/108944

(56) References cited:
- WO-A-96/40975
- JP-A- 1 225 496
- JP-A- 3 119 996
- JP-A- 4 234 871
- JP-A- 11 080 149
- JP-A- 11 206 398
- JP-A- 2002 167 381
- JP-A- 2003 144 190
- FERORELLI S. ET AL: 'Lipase-mediated kinetic resolution of rigid clofibrate analogues with lipid-modifying activity' TETRAHEDRON: ASYMMETRY vol. 12, no. 6, 2001, pages 853 - 862, XP004241707

## Description

### TECHNICAL FIELD

The present invention relates to a method for producing optically active chromancarboxylates, and more particularly to a method for producing 6-hydroxy-2,5,7,8-tetramethylchroman-2-carboxylic acid esters. The optically active chromancarboxylates are useful as the raw materials for medicines, agricultural chemicals, chiral building blocks and other functional chemical products, and for example, usable as the intermediates for the production of optically active vitamin E derivatives and anti-inflammatory agents.

### BACKGROUND ART

Methods for producing optically active carboxylic acids are roughly classified into three groups: an optical resolution utilizing diastereomeric salts, a hydrolysis or acylation using a biocatalyst, and a chiral pool method where the target compound is derived from known chiral building blocks. An asymmetric synthesis utilizing asymmetric ligands has been recently developed, but only a few has been reported for success. The optically active chromancarboxylic acids which are useful as the raw materials for medicines are produced, for example, by (1) a diastereomeric resolution using optically active amines (JP-A-11-80149 and WO 02/12221); (2) an enantiospecific hydrolysis of (±)-6-hydroxy-2,5,7,8-tetramethylchromancarboxylate using an enzyme catalyst (US Patent No. 5,348,973); (3) a halolactonization of optically active acylproline derivatives ("Chemistry Letters", p. 465 (1998)); or (4) a reaction of an organotitanium compound with an optically active pyruvic acid ester (EP-A-0173142 and JP-A-61-60628).

However, the method 1 requires a complicated crystallization procedure and produces a large amount of waste water in the acid/base treatment. The method 2 requires complicated procedures for the isolation and purification of the aimed product after the enantiospecific hydrolysis and for the removal of the enzyme. The methods 3 and 4 are less practicable because the starting optically active substances and the organotitanium compound are not easily available. Thus, these known methods are not necessarily advantageous for the industrial production of the optically active chromancarboxylic acid derivatives. Also known is a method of producing optically active chromancarboxylic acids by the hydrolysis of a racemic chromancarboxylate. However, there has been no report on a method of producing optically active chromancarboxylates by an enantiospecific esterification of one of R-and S-isomers of racemic chromancarboxylic acid by the use of a biocatalyst.

### DISCLOSURE OF THE INVENTION

An object of the present invention is to provide an efficient and industrially practicable method for producing optically active chromancarboxylates which are useful as the raw materials for medicines, agricultural chemicals, etc.

As a result of extensive study for achieving the above object, the inventors have found that the reaction of a racemic chromancarboxylic acid, for example, racemic 6-hydroxy-2,5,7,8-tetramethylchroman-2-carboxylic acid with methanol in an organic solvent in the presence of a biocatalyst allows only one of R- and S-isomers of the racemic mixture to be enantiospecifically and rapidly esterified. It has been further found that such a reaction less reduces the catalyst life and has a high enantiospecificity to produce the ester with an optically high purity. It has been also found that such a reaction facilitates the separation and recovery of the aimed product and can be used as a simple industrial process. The present invention has been accomplished on the basis of these findings.

Thus, the present invention provides a method for producing an optically active chromancarboxylate, comprising a step of esterifying a racemic chromancarboxylic acid in an organic solvent comprising an alcohol under water content of 0.5% by weight or lower in the presence of a biocatalyst, wherein the biocatalyst is an immobilized lipase being derived from microorganisms belonging to the genus *Candida,* and the chromancarboxylic acid is selected from the group consisting of chroman-2-carboxylic acid, 6-hydroxy-2,7,8-trimethyl-2-carboxymethylchroman, 6-hydroxy-2,7,8-trimethylchroman-2-yl-propionic acid and 6-hydroxy-2,5,7,8-tetramethylchroman-2-carboxylic acid.

The chromancarboxylic acid used as a substrate is preferably 6-hydroxy-2,5,7,8-tetramethylchroman-2-carboxylic acid. The alcohol is preferably methanol.

In the method of the present invention, only one enantionmer of the racemic chromancarboxylic acid is esterified by the action of the biocatalyst, and the other enantiomer remains unreacted. Therefore, in addition to the method for producing the optically active chromancarboxylates, the present invention also provides a method of separating, from a racemic chromancarboxylic acid, a mirror image of the optically active chromancarboxylic acid which is converted into the ester. By hydrolyzing the optically active chromancarboxylate, a corresponding optically active chromancarboxylic acid is obtained.

The biocatalyst usable in the present method may be derived from any origins without particular limitations as long as it is capable of enantiospecifically esterifying only one enantiomer of the racemic chromancarboxylic acid in an organic solvent in the presence of an alcohol. Examples of the biocatalyst having such ability include a hydrolase produced by microorganisms. Examples of preferred biocatalysts include hydrolases derived from microorganisms belonging to genus *Candida,* genus *Rhizopus,* genus *Mucor,* genus *Aspergillus,* genus *Alcaligenes* and genus *Pseudomonus,* with a lipase produced by *Candida antarctica* being more preferred because it allows the esterification to proceed smoothly, has a high enantiospecificity and provides the aimed product in high yields. The biocatalyst can be used in any forms such as free form, immobilized form and cells of microorganisms.

The preferred substrate used in the present method includes a chromancarboxylic acid represented by the following formula 1:

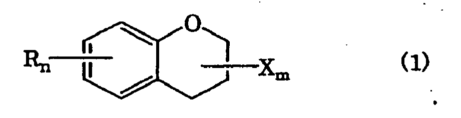

The term "chromancarboxylic acid" used herein means a compound having a chroman skeleton and at least one carboxyl group, such as chromancarboxylic acids in the strict sense, substituted chromancarboxylic acids, and substituted or unsubstituted chromans having a carboxyalkyl group.

In the present method, the racemic chromancarboxylic acid represented by the formula 1 is basically used as the substrate, but an optically active chromancarboxylic acid may be present in the reaction system. The production method of the present method is applicable to not only the chromancarboxylic acids represented by the formula 1 but also isochromancarboxylic acids and compounds having a chroman or isochroman ring having its oxygen atom replaced with another element of oxygen family such as sulfur, selenium and tellurium.

R in the formula 1 is a halogen atom, a hydroxyl group, a nitro group, an amino group, a cyano group, a chloromethyl group, a trifluoromethyl group, a carboxyl group, a carboxymethyl group, a carboxyethyl group, a carboxyphenyl group, a substituted or unsubstituted alkyl group or a substituted or unsubstituted aryl group.

The halogen atom is fluorine, chlorine, bromine or iodine.

Examples of the alkyl groups include linear, branched and cyclic alkyl groups having from 1 to 24 carbon atoms such as methyl, ethyl, n-propyl, isopropyl, n-butyl, isobutyl, t-butyl, n-amyl, isoamyl, n-hexyl, n-heptyl, 2-ethylhexyl, n-octyl and cyclohexyl.

Examples of the aryl groups include phenyl, benzyl, 2-methylbenzyl, 3-methylbenzyl, 4-methylbenzyl, 2-cumyl, 3-cumyl, 2-indenyl, 3-indenyl, 4-cumyl, 1-naphthyl, 2-naphthyl, biphenyl, phenyloxyphenyl, 2-methylphenyl, 3-methylphenyl, 4-methylphenyl, 2-ethylphenyl, 3-ethylphenyl, 4-ethylphenyl, 2,4-dimethylphenyl, 2,3-dimethylphenyl, 2,5-dimethylphenyl, 2,6-dimethylphenyl, 3,4-dimethylphenyl, 3,5-dimethylphenyl, 2,3,4-trimethylphenyl, 2,4,6-trimethylphenyl, 3,4,5-trimethylphenyl, 2,4,6-trimethylphenyl, 2,3,4,5-tetramethylphenyl, 2,3,4,6-tetramethylphenyl, 2,3,6,6-tetramethylphenyl, 2-fluorophenyl, 3-fluorophenyl, 4-fluorophenyl, 2-chlorophenyl, 3-chlorophenyl, 4-chlorophenyl, 2-trifluoromethylphenyl, 3-trifluoromethylphenyl, 4-trifluoromethylphenyl, 2-methoxyphenyl, 3-methoxyphenyl, 4-methoxyphenyl, 2-ethoxyphenyl, 3-ethoxyphenyl and 4-ethoxyphenyl.

Examples of the substituents for the alkyl or aryl groups include halogen atoms such as fluorine, chlorine, bromine and iodine, hydroxyl group, nitro group, amino group, chloromethyl group, carboxyl group, trifluoromethyl group, trichloromethyl group, methoxy group, ethoxy group, mercapto group, amido group, cyano group, carbonyl group, acetyl group, acyl groups, alkoxy groups, sulfone group and sulfonic acid group.

Preferred R groups are hydroxyl group, methyl group, carboxyl group, carboxymethyl group and carboxyethyl group.

The suffix m represents an integer of from 0 to 4. When m is an integer of from 2 to 4, R groups may be the same or different.

Like the R group, X is a halogen atom, a hydroxyl group, a nitro group, an amino group, a cyano group, a chloromethyl group, a trifluoromethyl group, a carboxyl group, a carboxymethyl group, a carboxyethyl group, a carboxyphenyl group, a substituted or unsubstituted alkyl group, or a substituted or unsubstituted aryl group, with a hydroxyl group, a methyl group, a carboxyl group, a carboxymethyl group and a carboxyethyl group being preferred. Specific examples of X group are the same as those exemplified for R, and therefore, omitted here for conciseness.

The suffix n is an integer of from 1 to 6. At least one of Xₙ is a carboxyl group, a carboxymethyl group, a carboxyethyl group or a carboxyphenyl group. When n is an integer of from 2 to 6, X groups may be the same or different. In addition, the same or different kinds of two X groups may be bonded to the same carbon atom.

Examples of the chromancarboxylic acids of the formula 1 include chroman-2-carboxylic acid, chroman-3-carboxylic acid, chroman-4-carboxylic acid, 6-hydroxychroman-2-carboxylic acid, 6-hydroxychroman-2-methyl-2-carboxylic acid, 2-carboxymethyl-6-hydroxy-2-methylchroman, 6-hydroxy-5-methylchroman-2-carboxylic acid, 6-hydroxy-7,8-dimethylchroman-2-carboxylic acid, 6-hydroxy-2,7,8-trimethyl-2-carboxymethylchroman, 6-hydroxy-2,7,8-trimethylchroman-2-carboxylic acid, 6-hydroxy-2,7,8-trimethylchroman-2-ylpropionic acid and 6-hydroxy-2,5,7,8-tetramethylchroman-2-carboxylic acid. Preferred are vitamin E derivatives, such as tocol, tocotrienol and a, p, y, δ, s or η-tocopherol, having a carboxyl group or a carboxymethyl group at the 2-position of their chroman ring, with chroman-2-carboxylic acid, 6-hydroxy-2,7,8-trimethyl-2-carboxymethylchroman, 6-hydroxy-2,7,8-trimethylchroman-2-ylpropionic acid and 6-hydroxy-2,5,7,8-tetramethylchroman-2-carboxylic acid being more preferred.

Alcohols having from 1 to 24 carbon atoms are suitably used for the reaction with the chromancarboxylic acid. Preferred examples of the alcohols include methanol, ethanol, n-propyl alcohol, isopropyl alcohol, glycidol, n-butyl alcohol, isobutyl alcohol, t-butyl alcohol, n-amyl alcohol, allyl alcohol, hexanol, n-octyl alcohol, 2-ethylhexyl alcohol, lauryl alcohol, stearyl alcohol, cyclohexanol, benzyl alcohol, ethylene glycol, 1,3-propanediol and 1,4-butanediol, with methanol, ethanol, n-propyl alcohol, isopropyl alcohol, n-butyl alcohol and isobutyl alcohol being more preferred, and methanol being still more preferred.

The solvents used in the esterification are preferably alcohols which also act as the esterifying agent. In addition to the alcohols, solvents other than alcohols may be used. The solvent is selected taking the boiling point, the dissolving power to the substrate, the degree of inhibition to the activity of the biocatalyst, the reaction temperature range, advantages in process, etc. into consideration. All suitable solvents are not mentioned here, but preferred are those well, dissolving the substrate and being hardly miscible with water. Examples of the solvents include, in addition to the alcohols having from 1 to 24 carbon atoms described above, aliphatic hydrocarbons such as n-hexane and n-heptane, aromatic hydrocarbons such as benzene, toluene and xylene, and ethers such as diethyl ether, diisopropyl ether, t-butyl methyl ether and n-dibutyl ether. These solvents may be used alone or in combination of two or more.

The enantiospecific esterification of the present method is conducted by bringing the racemic chromancarboxylic acid of the formula 1 into contact with the alcohol in the organic solvent in the presence of the biocatalyst. The esterification conditions vary depending upon the kinds of chromancarboxylic acids as the substrate. For example, in the esterification using 6-hydroxy-2,5,7,8-tetramethylchroman-2-carboxylic acid, the concentration of the chromancarboxylic acid in the reaction solution is preferably from 1 to 30% by weight and more preferably from 5 to 15% by weight. If a solvent other than the alcohol is used, the concentration of the alcohol as the esterifying agent in the reaction solution is from 1 to 20% by weight and preferably from 1 to 10% by weight. The molar ratio of the chromancarboxylic acid to the alcohol is preferably from 1:0.5 to 1:15 and more preferably from 1:1 to 1:5.

The amount of the biocatalyst to be used varies depending upon its activity to the esterification, and is preferably from 10 to 200% by weight and more preferably from 20 to 40% by weight of the weight of the chromancarboxylic acid as the substrate.

The esterification temperature is preferably from 30 to 60°C. The esterification can be conducted at temperatures higher than 60°C or lower than 30°C, if the biocatalyst is not deactivated, its life is not shortened, or the reaction rate is not adversely affected. The esterification may be carried out either under reduced pressure or under pressure of 0.1. MPa more, but preferably conducted under atmospheric pressure because of low apparatus costs.

Since a large amount of water reduces the reaction rate, it is preferred for an efficient esterification to remove water from the reaction system as much as possible. Therefore, the esterification is conducted preferably under a substantially water-free conditions (water content: 0.5% by weight or lower) by removing water out of the reaction system using a molecular sieve, etc.

In accordance with the method described above, one of R- and S-enantiomers of the racemic chromancarboxylic acid as the substrate is enantiospecifically converted into an optically active ester. Simultaneously, the mirror image of the chromancarboxylic acid which is converted into the optically active ester, i.e., the non-esterified enantiomer of chromancarboxylic acid, is obtained. By hydrolyzing the optically active ester, a corresponding optically active chromancarboxylic acid can be obtained. Since the separation and recovery of the reaction products are easy, the method of the present invention is suitable for industrial use.

After the esterification, the remaining non-reacted optically active chromancarboxylic acid can be transferred into the aqueous layer by the addition of sodium carbonate, because the alkali metal salt such as sodium salt of the optically active chromancarboxylic acid is highly soluble in water but less soluble in organic solvent. Thus, the optically active ester and the non-reacted optically active chromancarboxylic acid are easily separated from each other and recovered. During the separating operation, an organic solvent immiscible with water such as ethyl acetate may be added to prevent the optically active ester with a low solubility from being crystallized.

The optically active chromancarboxylate is isolated from the organic layer by distilling off the organic solvent under reduced pressure. The isolated optically active chromancarboxylate may be purified by recrystallization, etc. so as to attain desired chemical purity and optical purity.

The mirror image of the chromancarboxylic acid which is converted into the optically active ester can be obtained by the steps of neutralizing the aqueous layer containing an alkali metal salt of the non-reacted optically active chromancarboxylic acid with an aqueous solution of acid such as hydrochloric acid; separating the crystallized optically active chromancarboxylic acid by filtration or extraction with an organic solvent followed by the removal of solvent; and recrystallization. The optically active ester can be hydrolyzed into the corresponding optically active chromancarboxylic acid without causing racemization, for example, by dissolving the ester in methanol, adding an aqueous solution of sodium hydroxide and then heating.

As described above, the optically active chromancarboxylate can be easily produced by esterifying the racemic chromancarboxylic acid in the presence of the biocatalyst. By hydrolyzing the optically active ester, the corresponding optically active carboxylic acid can be produced. By separating the non-reacted optically active chromancarboxylic acid from the reaction solution after esterification, the mirror image of the chromancarboxylic acid which is esterified can be obtained.

The present invention will be described in more detail by reference to the following examples, but it should be noted that these examples are only illustrative and not intended to limit the scope of the present invention thereto.

In the following examples, the optical purity was determined by high performance liquid chromatography using "Sumichiral OA-3200" (4.6 φmm x 250 mm) available from Simika Chemical Analysis Service Co., Ltd.

### EXAMPLE 1

### (1) Production of racemic methyl 6-hydroxy-2,5,7,8-tetramethylchroman-2-carboxylate

In an autoclave, 20 g of trimethyl hydroquinone (TMHQ), 8 g of paraformaldehyde, 66 g of methyl methacrylate (MMA) and 4 g of acetic acid were stirred at 180°C for 3 h. After cooling, methanol was further added. The precipitates were colleted by filtration and washed, to obtain 25 g of the titled racemic methyl 6-hydroxy-2,5,7,8-tetramethylchroman-2-carboxylate (CCM).

### (2) Production of racemic 6-hydroxy-2,5,7,8-tetramethylchroman-2-carboxylic acid

A mixture of a solution of 25 g of CCM obtained above in 370 g of ethanol and 250 g of a 10 wt % aqueous solution of NaOH was stirred under reflux at 82 to 85°C for 2 h. Through the successive steps of concentration under reduced pressure, filtration, extraction, washing and recrystallization, 22 g of racemic 6-hydroxy-2,5,7,8-tetramethylchroman-2-carboxylic acid (CCA) was obtained.

### (3) Immobilization of enzyme

Into one milliliter of a phosphate buffer solution (pH 7.0), 20 mg of lipase derived from *Candida antarctica* ("Chirazyme L-2, lyo." available from Roche Diagnostics K. K.) was dissolved. After adding 0.4 mL of a carrier for immobilization "Toyonite 200-M," the solution was shaken over night at room temperature. Through filtration and air-drying, a biocatalyst for use in the following esterification was obtained.

### (4) Esterification

Into 2.5 g of t-butyl methyl ether, 50 mg of racemic 6-hydroxy-2,5,7,8-tetramethylchroman-2-carboxylic acid was dissolved. After adding 40 mg of methanol and 50 mg of the immobilized enzyme prepared above, the solution was shaken at 60°C for 24h. After the reaction, the reaction product solution was analyzed by HPLC. S-(-)-Methyl 6-hydroxy-2,5,7,8-tetramethylchroman-2-carboxylate having an optical purity of 99% ee or more was obtained in a 10.9% yield. The optical purity of the remaining non-reacted enantiomer (R-(+)-6-hydroxy-2,5,7,8-tetramethylchroman-2-carboxylic acid) was 69.4%.

### EXAMPLE 2

Into 58.8 g of isopropyl ether, 6 g of CCA and 4.2 g of methanol were dissolved. After adding 2 g of an immobilized enzyme ("Chirazyme L-2, c-f, C2" available from Roche Diagnostics K.K.) and purging the reaction system with argon, the reaction was conducted at 60°C for 24 h. The yield of S-(-)-CCM was 10 mol %.

### EXAMPLES 3-6

The procedure of Example 1 was repeated except for using 50 mg of respective immobilized enzyme, "Chirazyme L-2, c-f, C1," "Chirazyme L-2, c-f, C2," "Chirazyme L-2, c-f, C3" (all available from Roche Diagnostics K. K.) and "Novozyme 435" (available from Novozymes A/S). The yields of S-(-)-CCM were 10.8 mol %, 9.0 mol %, 10.4 mol % and 10.3 mol %, respectively.

### EXAMPLE 7

Into 50.40 g of isopropyl ether, 6 g of CCA and 3.6 g of methanol were dissolved. After adding 2 g of "Chirazyme L-2, c-f, C2" (available from Roche Diagnostics K. K.) and purging the reaction system with argon, the reaction was conducted at 60°C for 24 h. After the reaction, the reaction solution was discharged from the top of reaction vessel. The reaction was repeated under the same conditions after charging CCA, methanol and isopropyl ether in the same amounts as in the first run and purging the reaction container with argon. In such a manner, the reaction was repeated 10 times in total. The yield and the optical purity of the product were the substantially the same as those in the first run, and the activity and life of the catalyst were kept good.

After diluting the reaction product solution of the first run twice with ethyl acetate, the non-reacted chromancarboxylic acid was extracted into the aqueous layer with an aqueous solution of sodium carbonate. The organic layer was evaporated to dryness to obtain 0.7 g of crude crystals of optically active methyl chromancarboxylate (S-(-)-CCM). The yield was 10.5%, the chemical purity was 85.4%, and the optical purity was 96.1 %ee.

The enantiomeric chromancarboxylic acid in the aqueous layer was precipitated by the addition of hydrochloric acid, and the precipitates were collected by filtration and dried, to obtain 0.6 g of crude crystals of R-(+)-6-hydroxy-2,5,7,8-tetramethylchroman-2-carboxylic acid (chemical purity: 87%; optical purity: 96.5% ee).

### EXAMPLE 8

Into a mixture of 10 g of methanol and 2 g of water, 1.6 g of S-(-)-CCM (purity: 87%) produced in Example 7 was dissolved. After adding sodium hydroxide in an amount 4 times (molar basis) the S-(-)-CCM, the solution was stirred at 50°C for one hour. After the reaction, the reaction product solution was cooled and neutralized with a 5 N HCl. By further cooling the solution with ice, the chromancarboxylic acid was allowed to precipitate. The precipitates were collected by filtration and dried, to obtain 1.2 g of optically active S-(-)-CCA. The conversion was 100%., the yield was 96.7%, the chemical purity was 96.4%, and the optical purity was 97.8%ee.

### EXAMPLE 9

The esterification of CCA was conducted in the same manner as in Example 2 except for using non-immobilized "Chirazyme L-2" in an amount of 0.08 g which corresponded to immobilized enzyme catalyst used in Example 2 with respect to the activity. After the reaction at 60°C for 24 h, S-(-)-CCM having a chemical purity of 96.4% and an optical purity of 97.3%ee was obtained in a 6% yield.

### INDUSTRIAL APPLICABILITY

In accordance with the present invention, an optically active chromancarboxylate can be efficiently produced together with a mirror image of the chromancarboxylic acid converted into the optically active ester, both being useful as the materials for medicines, agricultural chemicals, etc. The optically active ester and the mirror image are easily separated from each other and recovered by a simple procedure, and the enzyme catalyst can be repeatedly used. Therefore, the method of the present invention is suitable for the industrial production of these compounds.

## Claims

1. A method for producing an optically active chromancarboxylate, comprising a step of esterifying a racemic chromancarboxylic acid in an organic solvent comprising an alcohol under water content of 0.5% by weight or lower in the presence of an immobilized lipase being derived from microorganisms belonging to the genus Candida, wherein the chromancarboxylic acid is selected from the group consisting of chroman-2-carboxylic acid, 6-hydroxy-2,7,8-trimethyl-2- carboxymethylchroman, 6-hydroxy-2,7,8-trimethylchroman-2-yl-propionic acid and 6-hydroxy-2,5,7,8-tetramethylchroman-2-carboxylic acid.

2. The method according to claim 11, wherein the alcohol is methanol, ethanol, n-propyl alcohol, isopropyl alcohol, n-butyl alcohol or isobutyl alcohol.

3. The method according to claim 2, wherein the alcohol is methanol.

4. The method according to any one of claims 1 to 3, wherein the chromancarboxylic acid is 6-hydroxy-2,5,7,8-tetramethylchroman-2-carboxylic acid.

5. The method according to any one of claims 1 to 4, further comprising a step of separating the mirror image of the chromancarboxylic acid which is converted into the optically active chromancarboxylate from the reaction production solution after the esterification.

6. The method according to any one of claims 1 to 5, further comprising a step of hydrolyzing the optically active chromancarboxylate.

## Patentansprüche

1. Verfahren zur Herstellung eines optisch aktiven Chromancarboxylats, umfassend einen Schritt der Veresterung einer racemischen Chromancarbonsäure in einem organischen Lösungsmittel, welches einen Alkohol enthält, mit einem Wassergehalt von 0,5 Gew.-% oder weniger, in Gegenwart einer immobilisierten Lipase, die aus Mikroorganismen stammt, die zur Gattung Candida gehören, wobei die Chromancarbonsäure ausgewählt wird aus der aus Chroman-2-carbonsäure, b-Hydroxy-2,7,8-trimethyl-2-carboxymethylchroman, 6-Hydroxy-2,7,8-trimethylchroman-2-yl-propionsäure und 6-Hydroxy-2,5,7,8-tetramethylchroman-2-carbonsäure bestehenden Gruppe.

2. Verfahren nach Anspruch 1, wobei der Alkohol Methanol, Ethanol, n-Propylalkohol, Isopropylalkohol, n-Butylalkohol oder Isobutylalkohol ist.

3. Verfahren nach Anspruch 2, wobei der Alkohol Methanol ist.

4. Verfahren nach einem der Ansprüche 1 bis 3, wobei die Chromancarbonsäure 6-Hydroxy-2,5,7,8-tetramethylchroman-2-carbonsäure ist.

5. Verfahren nach einem der Ansprüche 1 bis 4, darüber hinaus umfassend einen Schritt der Abtrennung des Spiegelbilds der Chromancarbonsäure, die in das optisch aktive Chromancarboxylat umgewandelt wird, aus der Reaktionsproduktlösung nach der Veresterung.

6. Verfahren nach einem der Ansprüche 1 bis 5, darüber hinaus umfassend einen Schritt der Hydrolyse des optisch aktiven Chromancarboxylats,

## Revendications

1. Procédé de production d'un chromanecarboxylate optiquement actif, comprenant une étape d'estérification d'un acide chromanecarboxylique racémique dans un solvant organique comprenant un alcool sous une teneur en eau de 0,5 % en poids ou inférieure en présence d'une lipase immobilisée dérivée de micro-organismes appartenant au genre Candida, dans lequel l'acide chromanecarboxylique est choisi dans le groupe constitué d'acide chromane-2-carboxylique, de 6-hydroxy-2,7,8-triméthyl-2-carboxyméthylchromane, d'acide 6-hydroxy-2,7,8-triméthylchroman-2-ylpropionique et d'acide 6-hydroxy-2,5, 7,8-tétraméthylchromane-2-carboxylique.

2. Procédé selon la revendication 1, dans lequel l'alcool est le méthanol, l'éthanol, l'alcool n-propylique, l'alcool isopropylique, l'alcool n-butylique ou l'alcool isobutylique.

3. Procédé selon la revendication 2, dans lequel l'alcool est le méthanol.

4. Procédé selon l'une quelconque des revendications 1 à 3, dans lequel l'acide chromanecarboxylique est l'acide 6-hydroxy-2,5,7,8-tétraméthylchromane-2-carboxylique.

5. Procédé selon l'une quelconque des revendications 1 à 4, comprenant en outre une étape consistant à séparer l'image en miroir de l'acide chromanecarboxylique qui est converti en chromanecarboxylate optiquement actif de la solution de production de la réaction après l'estérification.

6. Procédé selon l'une quelconque des revendications 1 à 5, comprenant en outre une étape consistant à hydrolyser le chromanecarboxylate optiquement actif.
